# EUROPÄISCHE PATENTSCHRIFT

(11) **EP 0 809 966 B1**
(45) Veröffentlichungstag und Bekanntmachung des Hinweises auf die Patenterteilung: **22.06.2005**
(21) Anmeldenummer: 97105614.8
(22) Anmeldetag: 04.04.1997
(51) Int. Cl.: A61B 5/0215, G01L 19/00

(54) **Durchflussmesszelle zur extrakorporalen Messung von Blutparametern**
Through-flow cell for extracorporeal measurement of blood parameters
Cellule d'écoulement destinée à la mesure extracorporelle de paramètres sanguins

(30) Priorität: 31.05.1996 DE 19622079
(43) Veröffentlichungstag der Anmeldung: 03.12.1997
(73) Patentinhaber: SPHERE Medical Limited, Cambridge Cambridgeshire CB2 5GG (GB)
(72) Erfinder: Abraham-Fuchs, Klaus, Dipl.-Phys., 91058 Erlangen (DE); Gumbrecht, Walter, Dr., 91074 Herzogenaurach (DE); Hierold, Christofer, Dr., 81739 München (DE)
(74) Vertreter: McGowan, Nigel George

(56) Entgegenhaltungen:
- WO-A-92/05449
- US-A- 4 554 927
- US-A- 4 608 996
- US-A- 4 625 560
- US-A- 5 086 777

## Beschreibung

Die Erfindung betrifft eine Durchflußmeßzelle zur extrakorporalen Messung von Blutparametern mit einem in einer Meßkammer angeordneten Drucksensor.

Eine Durchflußmeßzelle mit den Merkmalen des Oberbegriffs des Anspruchs 1 ist aus der PCT-Veröffentlichung WO 92/05449 bekannt. Die Durchflußmeßzelle umfaßt eine erste Meßkammer mit mehreren darin angeordneten elektrochemischen Sensoren. Eine Einlaßöffnung der ersten Meßkammer ist über eine Flüssigkeitsverbindungsleitung mit einer Schalteinheit verbunden. An einer Auslaßöffnung der ersten Meßkammer ist eine Flüssigkeitsfördereinrichtung angeschlossen. Je nach Schaltstellung der Schalteinheit können Meßflüssigkeit, Spüllösung oder Eichlösung mittels der Flüssigkeitsfördereinrichtung durch die Meßkammer hindurchgesaugt und dann in einen Verwurf befördert werden. Getrennt von der ersten Meßkammer ist ein Drucksensor in einer zweiten Meßkammer der Durchflußmeßzelle angeordnet. Die zweite Meßkammer ist über eine weitere Flüssigkeitsverbindungsleitung mit der Schalteinheit verbunden. In der Schalteinheit wird in den Schaltzuständen "Bereit" und "Messen" eine Verbindung zu einer Meßleitung hergestellt, so daß in diesen Schaltstellungen eine kontinuierliche Druckmessung erfolgen kann.

Eine weitere Durchflußmeßzelle zur extrakorporalen Messung von mehreren elektrochemisch gemessenen Blutparametern, wie z. B. pH-, pO₂-, pCO₂-Wert und Elektrolyt-Konzentration, ist in dem US-Patent 4,841,974 beschrieben. Sie wird an einen arteriellen Dauerkatheter angeschlossen. Die Sensoren sind in der Durchflußmeßzelle in einer Wandung eines Meßkanals angeordnet. Die Sensoren selbst sind als ionensensitive Feldeffekt-Transistoren ausgebildet, die gemeinsam auf einem Siliziumchip integriert sind. Dabei dient als Steuerelektrode eine Membran, die eine Grenzfläche zum Meßmedium, also zum entnommmenen Blut, bildet. Eine zugeordnete Auswerteelektronik ist ebenfalls auf dem Chip integriert und bildet eine Baueinheit mit den Sensoren. Der Aufbau des Chips ist auch beschrieben in dem Artikel von W. Gumbrecht, D. Peters, W. Schelter, W. Erhardt, J. Henkel, J. Steil und U. Sykora: "Integrated pO₂, pCO₂, pH Sensor System for Online Blood Monitoring", Sensors and Actuators B, 18-19 (1994), pp. 704-708.

In der EP-A-0 714 017 sind Silizium-Dünnfilm-Drucksensoren beschrieben, die kompatibel mit in der CMOS-Technologie üblichen Prozessen hergestellt werden können. Kompatibel zu CMOS-Herstellprozessen bedeutet, daß die bei der Herstellung des Sensors benötigten zusätzlichen Prozeßschritte in Standard-CMOS-Prozeßschritte eingebunden werden können und diese nicht stören. Diese Technologie wird auch als Oberflächen-Mikromechanik-Technologie bezeichnet. Zusätzlich zu dem Drucksensor sind dort auf demselben Chip noch elektronische Schaltungen monolithisch integriert.

In der US 4,625,560 ist ein kapazitiver digitaler Druckwandler in Form eines integrierten Schaltkreises beschrieben. Dieser Druckwandler umfasst einen aktiven und einen passiven Wandlerteil. Mit Hilfe des passiven Wandlerteils wird ein störender Einfluss der Umwelt, wie elektrisches, thermisches, inhärentes Rauschen usw., auf das Ausgangssignal kompensiert.

Demgegenüber steht eine zur Oberflächen-Mikromechanik verwandte Technologie, die auch als Bulk-Mikromechanik-Technologie bezeichnet wird. Dabei werden die Sensoren wesentlich tiefer in das Silizium-Trägermaterial eingeätzt als bei der Oberflächen-Mikromechanik-Technologie.

Der Erfindung liegt nun die Aufgabe zugrunde, eine kompakte und robuste Durchflußmeßzelle zur extrakorporalen Messung von Blutparametern anzugeben, mit der ein einfach handhabbares und störungsempfindliches Blutmonitoring-System aufgebaut werden kann.

Die Aufgabe wird durch die Merkmale des Anspruchs 1 gelöst. Dabei ist das Ausgangssignal des baugleichen, aber inaktiven Drucksensorteils in gleicher Weise wie der Blutdrucksensor von weiteren Einflußgrößen, wie z. B. Temperatur, Spannungen im Trägermaterial sowie herstellungsbedingte Variabilitäten bestimmt und kann zur Korrektur dieser Störeinflüsse herangezogen werden. Die patientennahe Anordnung des Drucksensors ermöglicht eine weitgehend ungedämpfte Messung des arteriellen Blutdruckes. Aus Gründen der Meßsicherheit muß die zu analysierende Blutprobe durch den gesamten Meßkanal und noch weiter in den zweiten Zuführkanal gefördert werden. Der kleine Querschnitt des zweiten Zuführkanals hält das zur Messung der Blutparameter benötigte Gesamtvolumen in der Durchflußmeßzelle so gering wie möglich.

Eine vorteilhafte Ausgestaltung zeichnet sich dadurch aus, daß in der Meßkammer zusätzlich mindestens ein elektrochemischer Sensor angeordnet ist. Die Anordnung eines oder mehrerer elektrochemischer Sensoren mit dem Drucksensor in einem Gehäuse vereinfacht zum einen die Handhabung der Sensoren und macht die Durchflußmeßzelle störunempfindlich. Zum anderen ist dadurch die Möglichkeit gegeben, zusätzlich eine Auswerteelektronik in demselben Gehäuse anzuordnen, in dem dann alle Sensoren zur Bestimmung von Blutparametern untergebracht sind.

Eine weitere vorteilhafte Ausgestaltung zeichnet sich dadurch aus, daß außerhalb der Meßkammer ein Außendrucksensor angeordnet ist zur Messung eines außerhalb des Meßkanals herrschenden äußeren Luftdrucks. Mit dem Außendrucksensor wird der äußere Luftdruck gemessen und kann dann von dem Druck abgezogen werden, der mit dem mit der arteriellen Blutsäule in Verbindung stehenden Drucksensor gemessen wird. Da der in der Meßkammer angeordnete Drucksensor eine Summe von Umgebungsluftdruck und arteriellem Blutdruck mißt, kann durch Differenzbildung der arterielle Blutdruck bestimmt werden. Die Kommunikation des Außendrucksensors mit dem umgebenden Luftdruck kann durch eine Bohrung im Gehäuse hergestellt werden. Diese Bohrung kann mit einer feuchtigkeitsundurchlässigen Membran abgeschlossen sein, die den Luftdruck überträgt, jedoch ein Eindringen von Spritzwasser, Luftfeuchtigkeit oder ähnliches verhindert.

Bei einer weiteren vorteilhaften Ausgestaltung umfaßt der Außendrucksensor einen aktiven und einen inaktiven Außendrucksensorteil, wobei der inaktive Außendrucksensorteil baugleich mit dem aktiven Außendrucksensorteil ausgebildet ist und der inaktive Außendrucksensorteil durch eine steife Abdeckung vom äußeren Luftdruck isoliert ist. So können auch die Meßwerte des Außendrucksensors von Störeinflüssen befreit werden.

Eine kostengünstige und kompakte Durchflußmeßzelle läßt sich gemäß einer weiteren vorteilhaften Ausgestaltung aufbauen, indem der Drucksensor bzw. die Drucksensoren mit ersten Signalverarbeitungsbauteilen verbunden sind und die Signalverarbeitungsbauteile mit dem Drucksensor bzw. den Drucksensoren monolithisch auf einem Chip integriert sind. Die Signalverarbeitungsbauteile können z.B. mit CMOS-Technologie und die Drucksensoren mit CMOS-kompatibler Oberflächen-Mikromechanik-Technologie hergestellt werden.

Eine besonders kompakte Durchflußmeßzelle, die damit auch zum Messen nur ein geringes Blutvolumen benötigt, läßt sich dann aufbauen, wenn gemäß einer weiteren, besonders vorteilhaften Ausgestaltung alle Sensoren monolithisch auf einem Chip integriert sind. Die heute zur Verfügung stehende Oberflächen-Mikromechanik-Technologie erlaubt unter Einsatz von CMOS-kompatiblen Prozeßschritten die monolithische Integration aller Sensoren.

Bei einer weiteren vorteilhaften Ausgestaltung sind auch die den Sensoren zugeordneten Signalverarbeitungsbauteile auf dem Chip monolithisch integriert. Damit wird zum einen die Störanfälligkeit der Durchflußmeßzelle mit den Sensoren weiter verringert, zum anderen kann das Meßsignal in eine störunempfindliche Form zum Übertragen umgeformt werden.

Die Erfindung wird im folgenden anhand von vier Figuren erläutert. Es zeigen:
- Fig. 1: in einer schematischen Schnittdarstellung eine Zwei weg-Durchflußmeßzelle mit Chemosensoren und einem Drucksensor,
- Fig. 2: eine Schnittdarstellung der Zweiweg-Durchflußmeßzelle nach Fig. 1 in einer Ebene II,
- Fig. 3: eine Schnittdarstellung der Zweiweg-Durchflußmeßzelle nach Fig. 1 in der Ebene III und
- Fig. 4: eine schematische Schittdarstellung einer Dreiweg-Durchflußmeßzelle mit Chemosensoren und einem Druck sensor.

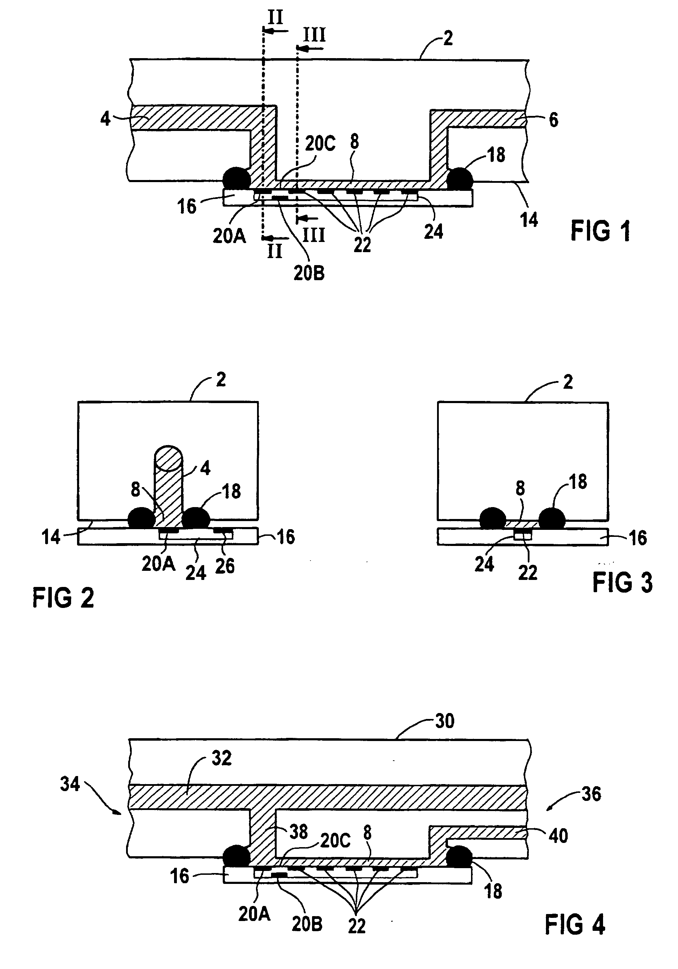

Die in Fig. 1 dargestellte Zweiweg-Durchflußmeßzelle wird eingesetzt zur extrakorporalen Messung von chemischen und physikalischen Blutparametern. In einem Trägerblock 2 sind Zuführkanäle 4 und 6 eingebracht, die jeweils zu einem Ende einer als Meßkanal 8 ausgebildeten Meßkammer führen. Die Zuführkanäle 4, 6 sind am anderem Ende jeweils mit Anschlußmitteln für Schlauchleitungen oder auch Katheter verbunden. Als Anschlußmittel kommen in der Medizintechnik übliche feste oder auch lösbare Verbindungselemente in Betracht; sie sind in Fig. 1 jedoch nicht dargestellt. Der erste Zuführkanal 4 ist vorgesehen zum Anschluß der Zweiweg-Durchflußmeßzelle an einen arteriellen Katheter. Der zweite Zuführkanal 6 ist unter anderem vorgesehen zum Anschluß der Zweiweg-Durchflußmeßzelle an eine Pumpe zum Fördern einer Blutprobe aus der Arterie zum Meßkanal 8. Die Zuführkanäle 4, 6 erstrecken sich zunächst in Längsrichtung in den Trägerblock 2 hinein, sie biegen dann zu einer Seitenfläche 14 hin und münden dort in den Meßkanal 8. Hier biegen die Zuführkanäle 4, 6 rechtwinklig zu der Seitenfläche 14 hin ab. Der Querschnitt des Zuführkanals 4 ist so groß ausgebildet, daß der arterielle Blutdruck nahezu ungedämpft zum Meßkanal 8 geleitet werden kann. Dagegen ist das Volumen des Meßkanals 8 und der Querschnitt des zweiten Zuführkanals 6 möglichst klein ausgebildet, um das benötigte Gesamtprobenvolumen in der Durchflußmeßzelle so gering wie möglich zu halten. Eine Wandung des Meßkanals 8 wird gebildet von einem Silizium-Halbleiterchip 16, das in einem Abstand, der die Höhe des Meßkanals 8 bestimmt, von der Seitenfläche 14 angeordnet ist. Ein umlaufender Dichtring 18 dichtet den Bereich zwischen dem Halbleiterchip 16 und dem Trägerblock 2 ab. Der Dichtring 18 begrenzt somit den Meßkanal 8 seitlich sowie die Mündungen der Zuführkanäle 4 und 6 in der Seitenfläche 14.

Auf dem Halbleiterchip 16 sind auf der Seite, die den Meßkanal 8 begrenzt, alle Sensoren integriert aufgebracht. In der Verlängerung des Zuführkanals 4 ist zunächst ein Drucksensor 20 angeordnet. Im Verlauf des Meßkanals 8 sind auf dem Halbleiterchip 16 weitere Sensoren 22 zur Messung von weiteren Blutparametern, wie z. B. Blutgaswerte, Elektrolyt- und Metabolit-Konzentrationen sowie Temperatursensoren und ggf. Strömungssensoren angeordnet. Die elektrochemischen Sensoren sind in Dünn- und Dickfilmtechnik ausgeführt, der Drucksensor ist in Oberflächen-Mikromechanik-Technologie ausgeführt. Ebenfalls integriert - vorzugsweise in CMOS-Technologie - auf dem Halbleiterchip 16 ist eine zu den Sensoren gehörende Signalverarbeitungsschaltung 24. Die Signalverarbeitungsschaltung 24 umfaßt Analog-Digitalwandler, die das Meßsignal digitalisieren, um eine störunempfindliche Signalübertragung vom Chip 16 zu weiteren elektronischen Auswertungsschaltungen zu ermöglichen.

Der Drucksensor umfaßt sowohl einen aktiven Drucksensorteil 20A, der ein vom Druck abhängiges Meßsignal abgibt, wie auch einen baugleichen inaktiven Drucksensorteil 20B, der durch eine steife Abdeckung 20C von dem im Meßkanal 8 herrschenden Druck isoliert ist. Das Ausgangssignal des baugleichen, jedoch inaktiven Drucksensorteils ist gleichermaßen wie das Ausgangssignal des aktiven Drucksensorteils von störenden Einflußgrößen, wie z. B. Temperatur, Verspannungen im Trägermaterial und herstellungsbedingte Toleranzen, bestimmt und kann zur Korrektur dieser Störeinflüsse herangezogen werden.

Fig. 2 zeigt in einer Schnittdarstellung entlang einer in Fig. 1 als II bezeichneten Schnittebene den ersten Zuführkanal 4, in dessen Verlängerung der aktive Drucksensorteil 20A auf dem Halbleiterchip 16 angeordnet ist. Zusätzlich zum Drucksensorteil 20A ist auf dem Halbleiterchip 16 noch ein Außendrucksensor 26 integriert zur Messung eines außerhalb des Meßkanals 8 herrschenden äußeren Luftdrucks. Der Außendrucksensor 26 umfaßt ebenfalls wie der Drucksensor im Meßkanal 8 einen aktiven Außendrucksensorteil und einen inaktiven Außendrucksensorteil zur Kompensation von störenden Einflußgrößen. Der Außendrucksensor 26 kann von der Umgebung mit einer feuchtigkeitsundurchlässigen Membran, die jedoch den Luftdruck überträgt, getrennt sein.

Der Blutdruckwert des Patienten ergibt sich dann prinzipiell, indem zunächst die von Störeinflüssen befreiten Druckmeßwerte des aktiven Drucksensorteils 20A und des Außendrucksensors 26 gebildet werden. Die von Störeinflüssen freien Druckmeßwerte ergeben sich z. B. aus einer Differenzbildung, wobei vom Meßwert des aktiven Drucksensorteils 20A der Wert des inaktiven Drucksensorteils 20B abgezogen wird. Den Blutdruckwert selbst erhält man dann durch eine weitere Differenzbildung, indem von dem ggf. von Störeinflüssen befreiten Blutdruckmeßwert der ggf. von Störeinflüssen befreite atmosphärische Außendruck abgezogen wird. Zumindest ein Teil dieser Signalverarbeitung wird durch auf dem Halbleiterchip 16 integrierte elektronische Signalverarbeitungsschaltkreise durchgeführt.

Die Schnittdarstellung in Fig. 3 entlang der Schnittebene III zeigt insbesondere den Querschnitt des Meßkanals 8, der im wesentlichen durch den Abstand des Halbleiterchips 16 vom Trägerblock 2 und der Größe der weiteren Sensoren 22 bestimmt ist.

Die in Fig. 4 im Schnitt dargestellte Dreiweg-Durchflußmeßzelle verwendet denselben Halbleiterchip 16 wie die Zweiweg-Durchflußzelle nach Fig. 1, lediglich die flüssigkeitsführenden Kanäle sind verändert. In einen Trägerblock 30 ist ein gerade verlaufender Durchflußkanal 32 mit einem großen Querschnitt eingearbeitet, der in den gegenüberliegenden linken und rechten Seiten 34 bzw. 36 mündet. Die Mündung in der linken Seite 34 ist zum Anschluß des arteriellen Dauerkatheters ausgebildet, während die Mündung in der rechten Seite 36 zum Anschluß eines Vorratsbehälters für eine Infusionslösung mit einer dazugehörigen Fördereinrichtung vorgesehen ist.

Vom Durchflußkanal 32 führt eine Abzweigung 38 zum Meßkanal 8. Der Querschnitt des Durchflußkanals 32 ist mindestens so groß wie der Querschnitt der Abzweigung 38, wobei der Querschnitt der Abzweigung 38 ausreichend groß ist, damit der zu messende arterielle Blutdruck ungedämpft bis zum Drucksensor 20 geleitet werden kann. Der Teil des Durchflußkanals 32 von der linken Seite 34 bis zur Mündung der Abzweigung 38 und dann bis zum Meßkanal 8 entspricht dem ersten Zuführkanal 4 der Zweiweg-Durchflußmeßzelle in Fig. 1. An das andere Ende des Meßkanals 8 ist ein Verbindungskanal 40 geführt, der ebenfalls in der rechten Seite 36 mündet. Der Verbindungskanal 40 ist vorgesehen zum Anschluß einer Blutfördereinrichtung, mit der Blutproben entnommen und zu den Sensoren 20A und 22 im Meßkanal 8 gepumpt werden können, er entspricht dem zweiten Zuführkanal 6 der Zweiweg-Durchflußmeßzelle in Fig. 1.

## Patentansprüche

1. Durchflußmeßzelle zur extrakorporalen Messung von Blutparametern mit einem in einer Meßkammer (8) angeordneten Drucksensor (20), und mit inem ersten Zuführkanal (4; 32, 38) der mit einem ersten Ende der Meßkammer (8) verbunden ist, wobei der erste Zuführkanal (4; 32, 38) zum Anschluß eines Katheters vorgesehen ist, **dadurch gekennzeichnet, daß** der Drucksensor (20) einen aktiven und einen inaktiven Drucksensorteil (20A bzw. 20B) umfaßt, daß der inaktive Drucksensorteil (20B) baugleich dem aktiven Drucksensorteil (20A) ausgebildet ist, daß der inaktive Drucksensorteil (20B) durch eine steife Abdeckung vom in der Meßkammer (8) herrschenden Druck isoliert ist, daß der Drucksensor (20) am ersten Ende der Meßkammer (8) angeordnet ist, und daß ein zweiter Zuführkanal (6; 40) mit einem zweiten Ende der Meßkammer (8) verbunden ist, daß der zweite Zuführkanal (6; 40) zum Anschluß einer Flüssigkeits-Fördereinrichtung vorgesehen ist, daß der erste und der zweite Zuführkanal (4; 32, 38 bzw. 6; 40) einen ersten bzw. zweiten Querschnitt aufweisen und daß der erste Querschnitt größer als der zweite Querschnitt ausgebildet ist.

2. Durchflußmeßzelle nach Anspruch 1, **dadurch gekennzeichnet, daß** in der Meßkammer (8) zusätzlich mindestens ein elektrochemischer Sensor (22) angeordnet ist.

3. Durchflußmeßzelle nach Anspruch 1 oder 2,
**dadurch gekennzeichnet, daß** außerhalb der Meßkammer (8) ein Außendrucksensor (26) angeordnet ist zur Messung eines außerhalb der Meßkammer (8) herrschenden äußeren Luftdrucks.

4. Durchflußmeßzelle nach Anspruch 3, **dadurch gekennzeichnet, daß** der Außendrucksensor (26) einen aktiven und einen inaktiven Außendrucksensorteil umfaßt, daß der inaktive Außendrucksensorteil baugleich mit dem aktiven Außendrucksensorteil ausgebildet ist und daß der inaktive Außendrucksensorteil durch eine steife Abdeckung vom äußeren Luftdruck isoliert ist.

5. Durchflußzelle nach einem der Ansprüche 1 bis 4, **dadurch gekennzeichnet, daß** der Drucksensor (20) bzw. die Drucksensoren mit ersten Signalverarbeitungsbauteilen (24) verbunden sind und daß die Signalverarbeitungsbauteile (24) mit dem Drucksensor (20) bzw. den Drucksensoren monolithisch auf einem Chip (16) integriert sind.

6. Durchflußzelle nach einem der Ansprüche 3 bis 5,
**dadurch gekennzeichnet, daß** der Außendrucksensor (26) bzw. die Außendrucksensoren mit zweiten Signalverarbeitungsbauteilen (24) verbunden sind und daß die Signalverarbeitungsbauteile (24) mit dem Außendrucksensor (26) bzw. den Außendrucksensoren monolithisch auf einem Chip (16) integriert sind.

7. Durchflußmeßzelle nach einem der Ansprüche 1 bis 6, **dadurch gekennzeichnet, daß** alle Sensoren (20, 22, 26) monolithisch auf einem Chip (16) integriert sind.

8. Durchflußmeßzelle nach Anspruch 7, **dadurch gekennzeichnet, daß** zusätzlich den Sensoren zugeordnete Signalverarbeitungsbauteile (24) auf dem Chip (16) integriert sind.

9. Durchflußmeßzelle nach einem der Ansprüche 5 bis 7, **dadurch gekennzeichnet, daß** der Chip oder die Chips (16) als Silizium-Chip ausgebildet ist.

## Claims

1. Through-flow cell for extra-corporeal measurement of blood parameters comprising a pressure sensor (20) arranged in a measuring chamber (8),
**characterised in that** the pressure sensor (20) has an active and an inactive pressure sensor component (20A or 20B), that the inactive pressure sensor component (20B) is identical in design to the active pressure sensor component (20A), that the inactive pressure sensor component (20B) is isolated from the pressure that is prevalent in the measuring chamber (8) by a rigid cover, and having a first feed channel (4; 32, 38) which is connected to a first end of the measuring chamber (8), the first feed channel (4; 32, 38) being provided for connection of a catheter;
that the pressure sensor (20) is arranged at the first end of the measuring chamber (8), and
that a second feed channel (6; 40) is connected to a second end of the measuring chamber (8),
that the second feed channel (6; 40) is provided for the connection of a fluid transport device,
that the first and the second feed channels (4; 32, 38 and 6; 40 respectively) have a first and a second cross section respectively and that the first cross section is designed to be larger than the second cross section.

2. Through-flow cell according to Claim 1,
**characterized in that** at least one electrochemical sensor (22) is additionally arranged in the measuring chamber (8).

3. Through-flow cell according to Claim 1 or 2,
**characterized in that** an external pressure sensor (26) is arranged outside the measuring chamber (8) to measure an external air pressure that is prevalent outside the measuring chamber (8).

4. Through-flow cell according to Claim 3,
**characterised in that** the external pressure sensor (26) comprises an active and an inactive external pressure sensor component,
that the inactive external pressure sensor component is identical in design with the active external pressure sensor component and that the inactive external pressure sensor component is isolated from the external air pressure by a rigid cover.

5. Through-flow cell according to one of Claims 1 to 4,
**characterised in that** the pressure sensor (20) or the pressure sensors are connected to the first signal-processing components (24) and
that the signal-processing components (24) are monolithically integrated on a chip (16) together with the pressure sensor (20) or the pressure sensors.

6. Through-flow cell according to one of Claims 3 to 5,
**characterised in that** the external pressure sensor (26) or external pressure sensors are connected to the second signal-processing components (24) and that the signal-processing components (24) are monolithically integrated on a chip (16) together with the external pressure sensor (26) or the external pressure sensors.

7. Through-flow cell according to one of Claims 1 to 6,
**characterised in that** all the sensors (20, 22, 26) are monolithically integrated on a chip (16).

8. Through-flow cell according to Claim 7,
**characterised in that** signal-processing components (24) which are additionally assigned to the sensors are integrated on the chip (16).

9. Through-flow cell according to one of Claims 5 to 7,
**characterised in that** the chip or chips (16) is/are configured as a silicon chip.

## Revendications

1. Cellule de mesure à flux continu pour la mesure extracorporelle de paramètres sanguins comportant un capteur de pression (20) agencé dans une chambre de mesure (8) et un premier canal d'amenée (4 ; 32, 38) qui est relié à une première extrémité de la chambre de mesure (8), le premier canal d'amenée (4 ; 32, 38) étant prévu pour le raccordement d'un cathéter, **caractérisée en ce que** le capteur de pression (20) comprend une partie active et une partie inactive de capteur de pression (20A et 20B), **en ce que** la partie inactive de capteur de pression (20B) est configurée de manière structurellement identique à la partie active de capteur de pression (20A), **en ce que** la partie inactive de capteur de pression (20B) est isolée par un revêtement rigide de la pression régnant dans la chambre de mesure (8), **en ce que** le capteur de pression (20) est agencé à la première extrémité de la chambre de mesure (8) et **en ce qu'**un second canal d'amenée (6 ; 40) est relié à une deuxième extrémité de la chambre de mesure (8), **en ce que** le second canal d'amenée (6 ; 40) est prévu pour le raccordement d'un dispositif de transport de liquides, **en ce que** les premier et second canaux d'amenée (4 ; 32, 38 et 6 ; 40) présentent une première et une seconde section et **en ce que** la première section est plus grande que la seconde section.

2. Cellule de mesure à flux continu selon la revendication 1, **caractérisée en ce que** dans la chambre de mesure (8) on agence en plus au moins un capteur électrochimique (22).

3. Cellule de mesure à flux continu selon la revendication 1 ou 2, **caractérisée en ce que** à l'extérieur de la chambre de mesure (8), un capteur de pression externe (26) est agencé pour la mesure d'une pression d'air extérieur régnant à l'extérieur de la chambre de mesure (8).

4. Cellule de mesure à flux continu selon la revendication 3, **caractérisée en ce que** le capteur de pression externe (26) comprend une partie active et une partie inactive de capteur de pression externe, **en ce que** la partie inactive de capteur de pression externe est réalisée de manière structurellement identique à la partie active de capteur de pression externe et **en ce que** la partie inactive de capteur de pression externe est isolée par un revêtement rigide de la pression de l'air extérieur.

5. Cellule à flux continu selon l'une quelconque des revendications 1 à 4, **caractérisée en ce que** le capteur de pression (20) ou les capteurs de pression sont reliés à des premiers modules de traitement de signaux (24) et **en ce que** les modules de traitement de signaux (24) sont intégrés de manière monolithique sur une puce (16) avec le capteur de pression (20) ou les capteurs de pression.

6. Cellule à flux continu selon l'une quelconque des revendications 3 à 5, **caractérisée en ce que** le capteur de pression externe (26) ou les capteurs de pression externe sont reliés à des seconds modules de traitement de signaux (24) et **en ce que** les modules de traitement de signaux (24) sont intégrés de manière monolithique sur une puce (16) avec le capteur de pression externe (26) ou les capteurs de pression externe.

7. Cellule de mesure à flux continu selon l'une quelconque des revendications 1 à 6, **caractérisée en ce que** tous les capteurs (20, 22, 26) sont intégrés de manière monolithique sur une puce (16).

8. Cellule de mesure à flux continu selon la revendication 7, **caractérisée en ce que**, en plus, les modules de traitement de signaux (24) associés aux capteurs sont intégrés sur la puce (16).

9. Cellule de mesure à flux continu selon l'une quelconque des revendications 5 à 7, **caractérisée en ce que** la puce ou les puces (16) sont réalisées sous la forme de puces au silicium.
